(19) Europäisches Patentamt
European Patent Office
Office européen des brevets

(11) Veröffentlichungsnummer: **0 037 800**
**B1**

(12) **EUROPÄISCHE PATENTSCHRIFT**

(45) Veröffentlichungstag der Patentschrift: **12.08.87**

(51) Int. Cl.⁴: **A 61 K 31/35**, C 07 D 311/62

(21) Anmeldenummer: **81810130.5**

(22) Anmeldetag: **31.03.81**

(54) **Verwendung von O-substituierten Derivaten von (+)-Cyanidanol-3 als Verbindungen mit immunomodulatorischen Eigenschaften.**

(30) Priorität: **03.04.80 CH 2679/80**

(43) Veröffentlichungstag der Anmeldung: **14.10.81 Patentblatt 81/41**

(45) Bekanntmachung des Hinweises auf die Patenterteilung: **12.08.87 Patentblatt 87/33**

(84) Benannte Vertragsstaaten: **CH DE FR GB LI LU SE**

(56) Entgegenhaltungen:
**EP-A-0 003 274**
**DE-A-2 740 346**
**FR-A-2 345 441**
**US-A-4 248 789**

**CHEMICAL ABSTRACTS, Band 90, 1979, Seite 89, Nr. 67141y, Columbus, Ohio, U.S.A., I. MUCSI et al.: "Antiviral activity of flavonoids"**

(73) Patentinhaber: **Zyma SA, Route de l'Etraz, CH- 1260 Nyon (CH)**

(72) Erfinder: **Courbat, Pierre, Dr., 20, Route de Divonne, CH- 1260 Nyon (CH)**
Erfinder: **Weith, André, Dr., Résidence les Pins B, CH- 1197 Prangins (CH)**
Erfinder: **Albert, Alban, Dr., 62, Av.de Gennecy, CH- 1249 Avully (CH)**

(74) Vertreter: **Meyer, Hans Rudolf, Patentabteilung der CIBA- GEIGY AG Postfach, CH- 4002 Basel (CH)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft die Verwendung von O-substituierten Derivaten von (+)-Cyanidanol-3 als Verbindungen mit immunomodulatorischen Eigenschaften, und auch die pharmazeutischen Zusammensetzungen mit dieser Wirksamkeit.

Es wurde gefunden, dass die O-substituierten Derivate von (+)-Cyanidanol-3, insbesondere die 3-O-substituierten Derivate von (+)-Cyanidanol der allgemeinen Formel I

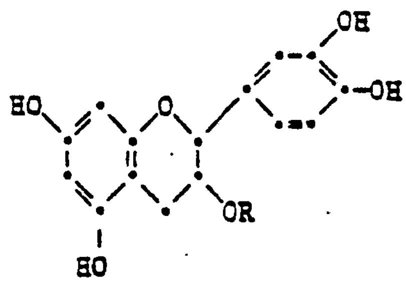

I

und ihre pharmazeutisch annehmbaren Salze, in welcher R einen Alkylrest mit 1 bis 4 Kohlenstoffatomen, der substituiert sein kann durch ein oder zwei Hydroxy-, Alkyloxy- mit 1 bis 4 Kohlenstoffatomen, Carboxy-, Alkanoyloxy- mit 1 bis 4 Kohlenstoffatomen, Amino-, Mono- oder Dialkylamino- mit 1 bis 4 Kohlenstoffatomen im Alkylteil oder Nitrilreste; einen Alkylrest mit 5 bis 14 Kohlenstoffatomen; einen Cykloalkylrest mit 5 bis 6 Ringkohlenstoffatomen, der substituiert sein kann durch ein oder zwei Hydroxy-, Alkyloxy- mit 1 bis 4 Kohlenstoffatomen, Carboxy-, Alkanoyloxy- mit 1 bis 4 Kohlenstoffatomen, Amino- oder Di-($C_1$-$C_4$)alkylaminoreste; einen Cycloalkanalkylrest, dessen Alkylteil 1 bis 4 Kohlenstoffatome, und dessen Ring 5 bis 6 Kohlenstoffatome aufweist; einen Phenylrest, der mono-, di- oder trisubstituiert sein kann durch Hydroxy-, Methoxy-, Äthoxy-, Propoxy-, Amino- oder Nitroreste; einen Benzyl-, Phenyläthyl- oder Phenylpropylrest, die am Benzolring mono- oder disubstituiert sein können durch ein oder zwei Hydroxy-, Äthoxy-, Methoxy-, Propoxy-, Carboxy-, Carbalkoxy- mit 1 bis 4 Kohlenstoffatomen, Nitro-, Amino- oder Mono- oder Dialkylaminoreste mit 1 bis 4 Kohlenstoffatomen im Alkylteil, oder durch Chlor- oder Fluoratome; einen Tetrahydropyranylrest; einen Pyridylrest, der substituiert sein kann durch einen Nitro-, Amino- oder Hydroxyrest; den Rest einer Alkancarbonsäure mit 3 bis 18 Kohlenstoffatomen, die substituiert sein kann durch einen oder mehrere Hydroxy-, Carboxy-, Carbalkoxy- mit 1 bis 6 Kohlenstoffatomen, Alkanoyl- mit 1 bis 5 Kohlenstoffatomen, Amino- oder Mono- oder Dialkylaminoreste mit 1 bis 4 Kohlenstoffatomen im Alkylteil oder durch Chlor- oder Fluoratome; den Rest einer Cycloalkancarbonsäure mit 5 bis 6 Ringkohlenstoffatomen, die substituiert sein kann durch 1, 2 oder 3 Hydroxy-, Carboxy-, Carbalkoxy- mit 1 bis 4 Kohlenstoffatomen, Amino- oder Mono- oder Dialkylaminoreste mit 1 bis 4 Kohlenstoffatomen oder durch Chlor- oder Fluoratome; den Rest einer Cycloalkan-Alkansäure, deren Alkylkette 2 bis 4 Kohlenstoffatome, und deren Cycloalkanteil 5 bis 6 Ringkohlenstoffatome enthält; einen benzoylischen Rest, der mono-, di- oder trisubstituiert sein kann durch Hydroxy-, Alkyloxy- mit 1 bis 4 Kohlenstoffatomen, Carboxy-, Carbalkoxy- mit 1 bis 4 Kohlenstoffatomen, Alkanoyl- mit 1 bis 4 Kohlenstoffatomen, Alkanoyloxy- mit 1 bis 4 Kohlenstoffatomen, Nitro-, Amino- oder Mono- oder Dialkylaminoreste mit 1 bis 4 Kohlenstoffatomen oder durch Chlor- oder Fluoratome; den Rest einer Phenylalkansäure, deren aliphatische Kette 1 bis 4 Kohlenstoffatome aufweist, und deren Phenylrest substituiert sein kann durch einen oder zwei Hydroxy-, Alkyloxy- mit 1 bis 4 Kohlenstoffatomen, Amino-, Carboxy-, Carbalkoxy- mit 1 bis 4 Kohlenstoffatomen, Alkanoyl- mit 1 bis 4 Kohlenstoffatomen oder Alkanoyloxyreste mit 1 bis 4 Kohlenstoffatomen oder Chlor- oder Fluoratome; einen Nikotinsäure- oder Isonikotinsäurerest; einen Alkyloxycarbonylrest mit 1 bis 4 Kohlenstoffatomen; einen Carbamoylrest, der monosubstituiert sein kann durch einen Alkylrest mit 1 bis 4 Kohlenstoffatomen oder einen Phenylrest; oder einen Rest einer nichtsubstituierten Phosphorsäure oder einer Dimethyl-, Diäthyl- oder Monohydroxyphenylphosphorsäure, bedeutet, immunomodulatorische Eigenschaften aufweisen, die ganz offensichtlich interessant sind für die Behandlung von Erkrankungen, bei denen eine Änderung der Ilmmunantwort des Organismus auftritt, wie bei sämtlichen rezidiven oder länger dauernden Viruserkrankungen, beispielsweise bei rezidiver Herpes, oder zur Behandlung von Erkrankungen aus der Gruppe bakterielle und parasitäre Viruserkrankungen, Krebserkrankungen und Autoimmunerkrankungen, wie beispielsweise rheumatische Polyarthritis.

Weiterhin ist zum Beispiel auch die Nützlichkeit dieser Verbindungen zur Behandlung von verschiedenen bösartigen Tumoren offensichtlich. Es handelt sich um Substanzen, die gleichzeitig eine erhöhte Antitumoraktivität und verringerte Sekundärwirkungen aufweisen. Der wirksame Bestandteil dieser neuen Produkte ist ein Biokatalysator, der nicht der Gruppe der Antitumorderivate des herkömmlichen Typus angehört. Da diese Substanzen eine direkte cytotoxische Wirksamkeit für Tumorzellen aufweisen, haben sie tatsächlich auch immunomodulatorische Eigenschaften, die in vivo nachgewiesen wurden, nicht nur in neoplastischen Modellen, sondern auch dank immunologischer Standarduntersuchungen. So beruht der ganze Wert dieser Verbindungen beim Kampf gegen den Krebs auf ihrer selektiven Toxizität gegenüber Krebszellen im Unterschied zu normalen Zellen, und auf ihrer Fähigkeit, das Abwehrpotential des Organismus zu

vermehren.

Beispielsweise sind diese Substanzen mit Erfolg vom nationalen Krebsinstitut der Vereinigten Staaten untersucht worden. Es handelt sich dabei um ein äusserst vollständiges Forschungs- bzw. Untersuchungsprogramm, das das leukämische System P388 bei der Maus verwendet. Behandlungen (kurzfristige oder länger andauernde) mit diesen Verbindungen ergeben einen direkten cytotoxischen Effekt auf diese leukämischen Zellen. Um diese Untersuchungsserie zu vervollständigen, sind auch zwei ascitische Tumore, das Sarkom 180 bei der Maus und das Hepatom AH13 bei Ratten als Modelle untersucht worden, um die direkte Antitumorwirksamkeit dieser Verbindungen zu zeigen. Die Verabreichung einer Dosis von 250 mg/ Kilogramm an Wirkstoff während 5 Tagen an Ratten und Mäuse reichte aus, um eine signifikante Verlängerung der Lebensdauer zu erreichen, verglichen mit Kontrolltieren, und eine Dosis von 500 mg/kg gewährleistet sogar ein Überleben von mehr als 30 Tagen bei 80 % der Tiere.

Diese Antitumorverbindungen haben gleichzeitig immunmodulatorische Eigenschaften, was in zahlreichen neoplastischen Modellen ermittelt worden ist.

So erlaubt die Leukämie L1210 Ha gemäss drei verschiedener Untersuchungsmethoden bei Mäusen,den Nachweis für diese wertvollen immunmodulatorischen Eigenschaften zu führen, so sind beispielsweise isogene Mäuse CD2F1 am Tag 0 mit $10^7$ bestrahlten Zellen L1210 Ha behandelt worden, und am 14. Tage wurden sie mit verschiedenen Mengen lebender Zellen mit der gleichen isogenen Leukämie geimpft. Die Wirksamkeit dieser Verbindungen zeigte sich an der verlängerten Lebensdauer und dem vergrösserten Prozentsatz an überlebenden Tieren nach 30 Tagen.

Darüber hinaus wurden Mäuse CD2Fl mit $10^5$ Zellen L1210 Ha geimpft, und am folgenden Tag wurden ihnen $10^7$ bestrahlte Tumorzellen injiziert. Die genannten Verbindungen wurden den Mäusen vor und nach der Impfung verabreicht. Die Wirksamkeit dieser Verbindungen ist äusserst positiv, indem die Lebensdauer vermehrt und der Prozentsatz an überlebenden Tieren nach 60 Tagen vergrössert wird. Darüber hinaus bestätigen Komplementärwirkungen bei Tieren, die zuvor mit 150 mg/kg Cyclophosphamid einer immunsuppressiven Behandlung unterzogen wurden, die beobachtete Wirksamkeit nach der Behandlung mit diesen Verbindungen, was anzeigt, dass die Reaktionsfähigkeit des Tieres in Ordnung ist.

Schliesslich lassen auch die Untersuchungsergebnisse von CD2F1-Mäusen, denen $10^5$ Zellen L1210 Ha transplantiert, und die am folgenden Tag mit Adriamycin behandelt wurden, die gleichen Schlüsse über die günstige Wirksamkeit dieser Verbindungen zu.

Diese Verbindungen haben nicht nur in bezug auf ascitische Tumore oder Leukämie eine günstige Wirksamkeit, sondern auch in bezug auf einen festen Tumor, das Carcinom von Lewis Lung (3LL) bei der Maus. Nun wird dieses neoplastische Modell gemäss E.O.R.T.C. als dasjenige angesehen, das menschliche Tumore am besten wiedergibt. Diese Substanzen zeigen bei drei Versuchsserien bedeutende positive Ergebnisse. Diese Verbindungen wurden während 10 Tagen an Mäuse C57B1/6 verabreicht, die mit dem isogenen Tumor 3LL infiziert waren. Sie wurden auch an Tiere mit Tumoren verabreicht, die zuvor einer Behandlung mit Methyl CCNU unterzogen wurden, bei einer Dosis von 10 mg/kg. Sie wirkten auf die gleiche Weise, indem sie die Bildung von Metastasen einschränkten, wenn die Primärgeschwulst chirurgisch entfernt worden ist.

Diese Verbindungen haben auch eine immunstimulatorische Wirksamkeit. So wurde ihr pharmazeutisches Potential anhand von in vivo-Versuchen nachgewiesen, das seiner Natur nach die cytotoxische Kapazität gereinigter Makrophagen im Hinblick auf Krebszellen vermehrt. Nun nimmt man an, dass diese Makrophagen, deren Kapazitäten durch diese Produkte merklich vergrössert worden sind, eine wesentliche Rolle spielen sowohl beim Kampf gegen Tumore, als auch bei der Kontrolle der immunologischen Reaktionsfähigkeit.

Schliesslich haben diese Verbindungen ihr therapeutisches Potential definitiv unter Beweis gestellt, indem sie auf unzweifelhafte Weise ihre positive Wirksamkeit bei der Erzeugung von Antikörpern unter nichtneoplastischen Bedingungen zeigen, was beweist, dass die Wirksamkeit dieser Produkte in der Tat auf die Reaktion des Wirtes zurückzuführen ist.

Bei der Injektion einer Maus CD2F1 mit $10^8$ Erythrocyten vom Schaf (SRBC) oder mit 0,5 $\mu$g polysacchariden Pneumokokken s.III wird die Anzahl der Splenocyten, die spezielle Antikörper erzeugen können, in deutlicher Weise vermehrt, was auch mit dem hämolytischen Plattentest auf Gel gemäss Jerne und Nordin gezeigt werden kann. Die Antikörper werden an den Ansprechspitzen gemessen, bei einmaligen oder wiederholten Injizierungen dieser Produkte.

Die Verbindungen der allgemeinen Formel I oder ihre Additionssalze sind in der europäischen Patentanmeldung EP-A- 003 274 oder in anderen entsprechenden Anmeldungen in anderen Ländern, die auf der schweizerischen Priorität vom 25. November 1977 basieren, beschrieben. Gemäss dieser Patentanmeldungen besitzen sie eine pharmakologische Wirksamkeit hinsichtlich der Verhütung hepatischer Nekrose, und sie inhibieren im übrigen die Lipoperoxidation. Sie können auch auf die Fibrillisation des Kollagens Einfluss nehmen oder auch den Abbau des Kollagens inhibieren sowie auch die Wirksamkeit von lysosomalen Enzymen, jeweils durch Vermehrung der Membranstabilität der Lysosomen.

Im vorangegangenen und auch im folgenden können die allgemeinen Bezeichnungen die folgende Bedeutung haben:

Ein Alkylrest mit 1 bis 14 Kohlenstoffatomen ist beispielsweise ein Methyl-, Äthyl-, n-Propyl-, Isopropyl-, n-Butyl-, Isobutyl-, sek.-Butyl- oder tert.-Butyl-, aber auch ein Pentyl-, Isopentyl-, n-Hexyl-, Isohexyl-, n-Heptyl-, Octyl-, Nonyl-, Decyl-, Undecyl-, Dodecyl-, Tricosyl-, Tetracosylrest und ihre Isomeren.

Substituierte Alkylreste mit 1 bis 4 Kohlenstoffatomen R sind in erster Linie Hydroxy- oder $C_1$-$C_4$-Alkyloxy-$C_1$-$C_4$-alkylreste, in denen die Hydroxyreste oder die $C_1$-$C_4$-Alkyloxyreste vorzugsweise durch wenigstens 2

Kohlenstoffatome von dem Sauerstoffatom getrennt sind, das den auf diese Weise substituierten Rest R trägt, wie beispielsweise der 2-Hydroxy-äthyl-, 2-Hydroxypropyl-, 3-Hydroxypropyl-, 2,3-Dihydroxy-propyl-, 2-Methoxyäthyl-, 2-Äthoxyäthyl-, 2-Methoxypropyl-, 3-Methoxypropyl- oder der 3-Äthoxypropylrest, sowie auch die Hydroxymethylreste.

Ein Cycloalkylrest mit 5 bis 6 Kohlenstoffatomen ist beispielsweise ein Cyclopentyl- oder Cyclohexylrest. Ein Cycloalkanalkylrest ist beispielsweise ein Cyclopentyl- oder Cyclohexylmethylrest, ein -1,1- oder -1,2-Äthylrest, ein -1,1-, -1,2- oder -1,3-Propylrest.

Ein Phenyläthylrest ist ein 1- oder 2-Phenyläthylrest, ein Phenylpropylrest ein 1-, 2- oder 3-Phenylpropylrest. Die am Kern substituierten Benzylreste tragen den Substituenten vorzugsweise in Para-Position.

Pyridylreste sind beispielsweise 2-Pyridyl-, 3-Pyridyl- oder 4-Pyridylreste.

Die Acylreste von Alkancarbonsäuren sind insbesondere Propion-, Butter-, Isobutter- und Valeriansäure sowie die höheren Homologen bis zur Stearinsäure, es sind weiterhin die Alkandicarbonsäuren, die beispielsweise 2 bis 10, vorzugsweise 3 bis 6 Kohlenstoffatome aufweisen, beispielsweise die 2-Methylbernsteinsäure, Glutarsäure, 3-Methylglutarsäure, 3-Äthylglutarsäure, Adipinsäure, Pimelinsäure, Suberinsäure, Azelainsäure oder Sebacinsäure, vorzugsweise die Malon- und Bernsteinsäuren.

Acylreste von Cycloalkancarbonsären mit 5 bis 6 Kohlenstoffatiomen sind in erster Linie solche von Cyclohexancarbonsäuren. Benzoesäurereste können beispielsweise durch einen Methoxy- oder Äthoxyrest oder durch einen Carboxyrest substituiert sein, unter diesen sind insbesondere zu nennen die Reste von Phthalsäure, Isophthalsäure oder Terephthalsäure.

Ein Rest einer substituierten Cycloalkancarbonsäure mit 5 bis 6 Ringkohlenstoffatomen ist derjenige der 1,2-Cyclohexandicarbonsäure.

Ein Alkoxycarbonylrest mit 1 bis 4 Kohlenstoffatomen ist beispielsweise ein Methoxycarbonyl-, Äthoxycarbonyl-, n-Propyloxycarbonyl-, Isopropyloxycarbonyl- oder tert.-Butyloxycarbonylrest.

Die gegebenenfalls durch einen Alkylrest mit 1 bis 4 Kohlenstoffatomen oder einen Phenylrest N-substituerten Carbamoylreste sind beispielsweise die N-Methyl-, N-Äthyl-, oder N-Phenyl-carbamoylreste.

Ein Alkoxyrest mit 1 bis 4 Kohlenstoffatomen ist beispielsweise, ein Methoxy-, Äthoxy-, n-Propyloxy-, Isopropyloxy-, n-Butyloxy-, Isobutyloxy-, sek.-Butyloxy-, oder tert.-Butyloxyrest.

Ein Mono- oder Dialkylaminorest mit 1 bis 4 Kohlenstoffatomen im Alkylteil ist beispielsweise ein Methylamino-, Dimethylamino-, Äthylamino- oder Diäthylaminorest.

Die Verbindungen der Erfindung, die einen salzbildenden Rest tragen, können auch in Form ihrer Salze vorliegen.

Die Salze von Verbindungen, die eine freie Carboxylgruppe aufweisen, sind beispielsweise die Metallsalze, insbesondere die Alkalimetallsalze, beispielsweise die Natrium- oder Kaliumsalze, sowie auch die Erdalkalimetallsalze, wie beispielsweise die Magnesium- oder Calciumsalze, sowie auch die Übergangsmetallsalze, beispielsweise die Zink-, Kupfer-, Eisen-, Silber- oder Quecksilbersalze, oder auch die Ammoniumsalze, wie beispielsweise die von Ammoniak oder organischen Basen, wie von Tri-Niederalkylaminen, beispielsweise von Trimethylamin oder Triäthylamin, insbesondere die nichttoxischen, pharmazeutisch einsetzbaren Salze der oben beschriebenen Art.

Die neuen Verbindungen, die basische Gruppen aufweisen, können auch in Form ihrer Additionssalze mit Säuren vorliegen, insbesondere in Form von nicht-toxischen, pharmazeutisch einsetzbaren Salzen, beispielsweise als Additionssalze mit Mineralsäuren, wie Chlorwasserstoffsäure, Bromwasserstoffsäure, Schwefelsäure oder Phosphorsäure, oder mit Carbonsäuren oder organischen Sulfonsäuren, beispielsweise aliphatischen, cycloaliphatischen, cycloaliphatisch-aliphatischen, aromatischen, araliphatischen, heterocyclischen oder heterocyclisch-aliphatischen Säuren, wie beispielsweise Essigsäure, Propionsäure, Bernsteinsäure, Glycolsäure, Milchsäure, Äpfelsäure, Weinsäure, Zitronensäure, Ascorbinsäure, Maleinsäure, Phenylessigsäure, Benzoesäure, 4-Amino-benzoesäure, Anthranilsäure, 4-Hydroxy-benzoesäure, Salicylsäure, Aminosalicylsäure, Embonsäure oder Nikotinsäure, wie auch Methansulfonsäure, Äthansulfonsäure, 2-Hydroxyäthansulfonsäure, Phenylsulfonsäure, 4-Methylphenylsulfonsäure, Naphthalinsulfonsäure, Sulfanilsäure oder Cyclohexylsulfaminsäure.

Die neuen Verbindungen, die basische Gruppen aufweisen, können auch in Form ihrer quaternären Ammoniumverbindungen vorliegen.

Erfindungsgemäss werden die Verbindungen der allgemeinen Formel I und ihre pharmazeutisch annehmbaren Salze als pharmazeutische Zusammensetzungen mit immunomodulatorischen Eigenschaften in solchen Dosen eingesetzt, die notwendig sind, um die gewünschte Wirkung bei Warmblütlern hervorzurufen. Es sind Zusammensetzungen, die den pharmakologisch wirksamen Bestandteil alleine oder in Mischung mit einem anderen aktiven Bestandteil der gleichen Wirkungsrichtung enthalten, zusammen mit wenigstens einem mineralischen oder organischen Trägermaterial, das fest oder flüssig und in Arzneimitteln verwendbar ist und das sich zur enteralen oder parenteralen Verabreichung eignet. Die Dosierung an aktivem Bestandteil hängt von der Tierart, dem Alter und dem individuellen Zustand des Tieres sowie auch von der Verabreichungsart ab. Vorzugsweise liegen diese pharmazeutischen Zusammensetzungen beispielsweise in Form einheitlicher Dosierungen vor, wie beispielsweise in Form von Dragees, Tabletten, Kapseln, Suppositorien und Ampullen.

Die einheitlichen Dosierungen zur peroralen Verabreichung enthalten als pharmakologisch wirksamen Bestandteil bis zu 99 %, vorzugsweise zwischen 1 bis 50 % einer Verbindung der allgemeinen Formel I oder eines pharmakologisch wirksamen Salzes davon. Zu ihrer Herstellung kombiniert man die Verbindungen zur Herstellung von Tabletten oder Drageekernen beispielsweise mit einem festen oder pulverförmigen

Trägermaterial, wie beispielsweise Lactose, Dextrose, Saccharose, Manit, Sorbit, Cellulose und/oder Stärke, wie beispielsweise Maisstärke, Weizenstärke, Reisstärke oder Marantastärke, und Gelatine, sowie, zusammen mit Gleitmitteln, falls gewünscht, wie beispielsweise Magnesiumstearat oder Calciumstearat, oder Polyäthylenglycol.

Die Kerne für die Dragees werden anschliessend beispielsweise mit einer konzentrierten Zuckerlösung überzogen, der man beispielsweise Gummi arabicum, Talk und/oder Titanoxid beigemischt hat, oder sie werden mit einem Lack überzogen, der in einem Lösungsmittel oder in einer Mischung von organischen, leicht verdampfbaren Lösungsmitteln gelöst ist.

Als andere einheitliche Dosierungen gemäss der Erfindung verwendet man beispielsweise Gelatinekapseln, die den Wirkstoff vorzugsweise in granulierter Form enthalten, beispielsweise in Mischung mit Verdünnungsmitteln, wie Maisstärke, und/oder Gleitmitteln, wie beispielsweise Talk, Magnesiumstearat, und, falls gewünscht, in Mischung mit Stabilisatoren, wie Natriumbisulfit ($Na_2S_2O_5$) oder Ascorbinsäure. In den weichen Kapseln ist der Wirkstoff vorzugsweise in geeigneten Flüssigkeiten gelöst oder suspendiert, wie in einem flüssigen Polyäthylenglycol, gegebenenfalls unter Zusatz eines Stabilisators.

Als erfindungsgemässe einheitliche Dosierungen zur rektalen Verabreichung kommen in erster Linie Suppositoria in Frage, die sich insbesondere aus einer Mischung einer primären Suppositoriummasse mit dem Wirkstoff zusammensetzen. Als primäre Suppositoriummasse sind beispielsweise zu nennen natürliche oder synthetische Triglycerine, Paraffinkohlenwasserstoffe, Polyäthylenglycole oder höhere Alkohole. Zu erwähnen sind hier auch Kapseln für die rektale Anwendung.

Ampullen zur parenteralen Verabreichung, insbesondere zur intravenösen Anwendung, enthalten vorzugsweise die wirksame Substanz oder ein Salz dieser wirksamen Substanz in Lösung, vorzugsweise in einer Konzentration von 0,5 bis 10 %.

Die folgenden Substanzen gemäss der vorliegenden Erfindung werden in einer Dosis von 1 bis 1000 mg, vorzugsweise 1 bis 500 mg pro Dosierungseinheit verabreicht.

Die vorliegende Erfindung betrifft als Wirkstoffe in erster Linie Verbindungen der allgemeinen Formel I, in welcher R bedeutet: einen Alkylrest mit 1 bis 4 Kohlenstoffatomen, der substituiert sein kann durch einen Hydroxy-, Alkyloxy- mit 1 bis 4 C-Atomen, Amino-oder Nitrilrest; einen Cycloalkanalkylrest, dessen Alkylteil 1 bis 4 Kohlenstoffatome, und dessen Ring 5 bis 6 Kohlenstoffatome umfassen; einen Benzyl-, Phenyläthyl- oder Phenylpropylrest, die nicht substituiert sind; einen Tetrahydropyranylrest; einen Rest einer Alkancarbonsäure mit 3 bis 18 Kohlenstoffatomen, die substituiert sein kann durch einen Hydroxy- oder Carboxyrest; einen Rest einer Cycloalkancarbonsäure mit 5 bis 6 Ringkohlenstoffatomen, die substituiert sein kann durch einen benzoylischen Rest, welcher seinerseits mono-, di- oder trisubstituiert sein kann durch Hydroxy-, Carboxy-, Carbalkoxy- mit 1 bis 4 Kohlenstoffatomen oder Alkanoyloxyreste mit 1 bis 4 Kohlenstoffatomen, oder durch Chlor- oder Fluoratome; einen Rest einer Phenylalkansäure, deren aliphatische Kette 1 bis 4 Kohlenstoffatome umfasst, und deren Phenylrest nicht substituiert ist; einen Rest einer Nikotin- oder Isonikotinsäure; einen Alkyloxycarbonylrest mit 1 bis 4 Kohlenstoffatomen; einen Carbamoylrest, der monosubstituiert ist durch Alkylreste mit 1 bis 4 Kohlenstoffatomen oder einen Phenylrest; oder einen Rest einer nichtsubstituierten Phosphorsäure oder einer Dimethyl-, Diäthyl- oder Monohydroxyphenylphosphorsäure.

Als Wirkstoff kommen in erster Linie in Betracht diejenigen Verbindungen der allgemeinen Formel I, bei denen R die folgende Bedeutung hat: einen Alkancarbonsäurerest mit 3 bis 18 Kohlenstoffatomen, der substituiert sein kann durch einen Hydroxy- oder Carboxyrest; einen Rest einer Cycloalkancarbonsäure mit 5 bis 6 Ringkohlenstoffatomen, die substituiert sein kann durch einen Carboxyrest; oder einen benzoylischen Rest, der mono-, di- oder trisubstituiert sein kann durch Hydroxy-, Alkyloxy- mit 1 bis 4 Kohlenstoffatomen, Carboxy-, Carbalkoxy- mit 1 bis 4 Kohlenstoffatomen oder Alkanoyloxyreste mit 1 bis 4 Kohlenstoffatomen, oder durch Chlor- oder Fluoratome; einen Rest einer Phenylalkansäure, deren aliphatische Kette 1 bis 4 Kohlenstoffatome umfasst, und deren Phenylrest nichtsubstituiert ist; einen Rest einer Nikotin- oder Isonikotinsäure; einen Alkyloxycarbonylrest mit 1 bis 4 Kohlenstoffatomen; einen Carbamoylrest, der monosubstituiert ist durch Alkylreste mit 1 bis 4 Kohlenstoffatomen oder den Phenylrest; oder einen Rest einer nichtsubstituierten Phosphorsäure oder Dimethyl-, Diäthyl- oder Monohydroxyphenylphosphorsäure.

Die Erfindung wird in den folgenden Beispielen weiter erläutert, die jedoch keine Einschränkung darstellen, und in welchen die Temperaturen in Grad Celsius angegeben sind.

**Beispiel 1:**

Zur Herstellung von 15.000 Tabletten werden 3,75 kg 3-0-Palmitoyl-(+)-cyanidanol-3 mit 0,18 kg Siliciumdioxid und 0,075 kg Getreidestärke gemischt. Nach der feuchten Granulierung mit 0,12 kg Getreidestärke und 0,225 kg Gelatine wird das Granulat trocken mit 0,03 kg Magnesiumstearat gemischt. Anschliessend werden auf einer Rotationsmaschine 15.000 Tabletten hergestellt. Eine Tablette wiegt 295 mg und enthält 250 mg Wirkstoff.

**Beispiel 2:**

Die gemäss Beispiel 1 hergestellten Tabletten werden schliesslich mit einer Lösung auf der Basis von Methylenchlorid überzogen, die 1,35 kg Polyvinylpyrolidon, 6,3 kg Methylcellulose, 1,4 kg Farbstoff und 3,24 kg Gummilack enthält.

**Beispiel 3:**

Zur Herstellung von Zuckerdragees werden die gemäss Beispiel 1 hergestellten Tabletten mit einem konzentrierten Sirup überzogen, der 20,8 kg Saccharose, 0,3 kg Schellack, 0,5 kg Gummi arabicum und 0,08 kg Farbstoff enthält. Nach dem Trocknen wiegen die Dragees jeweils 400 mg.

**Beispiel 4:**

Zur Herstellung von 1000 Kapseln, die 500 mg 3-O-Benzoyl-(+)-cyanidanol-3 enthalten, vermischt man 500 g 3-O-Benzoyl-(+)-cyanidanol-3 mit 8 g Stearinpulver und 1 g Magnesiumstearat. Die Mischung wird durch ein Netz mit einem Maschendurchmesser von 1 mm kalibriert und dann in 1000 Kapseln aus harter Gelatine in der Grösse Nr. 0 gefüllt.

**Beispiel 5:**

Es wird eine Granulierung auf der Basis von Wasser mit 1 kg 3-O-Heptyl-(+)-cyanidanol-3 und 2 kg Mannit durchgeführt. Nach dem Trocknen und der Kalibrierung wird das Granulat mit 100 g Fruchtaroma gemischt und in 1000 Säckchen abgefüllt. Jedes Säckchen enthält 1 g Wirkstoff.

**Beispiel 6:**

2 kg 3-O-Methoxymethyl-(+)-cyanidanol-3 werden in 6,0 kg Propylenglycol gelöst. Diese Mischung wird in eine Lösung gegeben, die 30,0 kg Saccharose, 3,0 kg Sorbit, 0,06 kg Natriumbisulfit, 0,06 kg Natriumbenzoat und 73 kg destilliertes Wasser enthält. Der auf diese Weise erhaltene Sirup enthält 2 % Wirkstoff.

**Beispiel 7:**

75 g 3-O-Methyl-(+)-katechin, 1,6 g Dinatriumphosphat und 34,5 g Natriumchlorid werden in 4,9 kg doppelt destilliertes Wasser gegeben. Nach der sterilen Filtration durch einen Membranfilter (Porendurchmesser: 0,2 µm) wird die Lösung unter Stickstoff in 1000 sterile Ampullen von je 5 ml abgefüllt. Jede Ampulle enthält 75 mg Wirkstoff in Form einer 1,5 %igen wässrigen Lösung.

**Beispiel 8:**

Man bereitet eine Suppositoriummasse zu, die 0,3 kg 3-O-Acetylsalicylyl-(+)-cyanidanol-3 und 1,7 kg Fettsäuretriglyceridäther enthält, und man formt daraus 1000 Suppositoria mit je 300 mg Wirkstoff.

**Beispiel 9:**

Zur Herstellung einer Salbe mit 5 % Wirkstoff werden 5 g 3-O-Dodecyl-(+)-cyanidanol-3 mit 10 g Propylenglycol und 10 g Wasser gemischt und dann emulgiert in einer 65 g Vaseline und 10 g Cetylstearylalkohol enthaltenden geschmolzenen Fettphase.

**Patentansprüche**

1. Verwendung eines 3-O-substituierten Derivats von (+)-Cyanidanol-3 der allgemeinen Formel

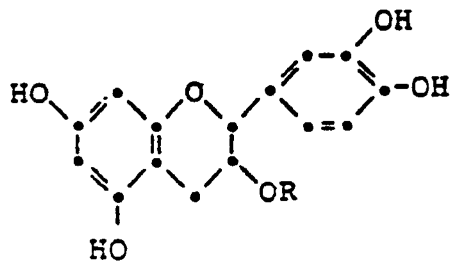

I

oder deren pharmazeutisch annehmbaren Salzen, in welcher R einen Alkylrest mit 1 bis 4 Kohlenstoffatomen der substituiert sein kann durch ein oder zwei Hydroxy-, Alkyloxy- mit 1 bis 4 Kohlenstoffatomen, Carboxy-, Alkanoyloxy- mit 1 bis 4 Kohlenstoffatomen, Amino-, Mono- oder Dialkylamino- mit 1 bis 4 Kohlenstoffatomen im Alkylteil oder Nitrilreste; einen Alkylrest mit 5 bis 14 Kohlenstoffatomen; einen Cycloalkylrest mit 5 bis 6 Ringkohlenstoffatomen, der substituiert sein kann durch ein oder zwei Hydroxy-, Alkyloxy- mit 1 bis 4 Kohlenstoffatomen, Carboxy-, Alkanoyloxy- mit 1 bis 4 Kohlenstoffatomen, Amino- oder Di-($C_1$-$C_4$)alkylaminoreste; einen Cycloalkanalkylrest, dessen Alkylteil 1 bis 4 Kohlenstoffatome, und dessen Ring 5 bis 6 Kohlenstoffatome aufweist; einen Phenylrest, der mono-, di- oder trisubstituiert sein kann durch Hydroxy-, Methoxy-, Äthoxy-, Propoxy-, Amino- oder Nitroreste; einen Benzyl-, Phenyläthyl- oder Phenylpropylrest, die am Benzolring mono- oder disubstituiert sein können durch ein oder zwei Hydroxy-, Äthoxy-, Methoxy-, Propoxy-, Carboxy-, Carbalkoxy- mit 1 bis 4 Kohlenstoffatomen, Nitro-, Amino- oder Mono- oder Dialkylaminoreste mit 1 bis 4 Kohlenstoffatomen im Alkylteil, oder durch Chlor- oder Fluoratome; einen Tetrahydropyranylrest; einen Pyridylrest, der substituiert sein kann durch einen Nitro-, Amino- oder Hydroxyrest; den Rest einer Alkancarbonsäure mit 3 bis 18 Kohlenstoffatomen, die substituiert sein kann durch einen oder mehrere Hydroxy-, Carboxy-, Carbalkoxy- mit 1 bis 6 Kohlenstoffatomen, Alkanoyl- mit 1 bis 5 Kohlenstoffatomen, Amino- oder Mono- oder Dialkylaminoreste mit 1 bis 4 Kohlenstoffatomen im Alkylteil oder durch Chlor- oder Fluoratome; den Rest einer Cycloalkancarbonsäure mit 5 bis 6 Ringkohlenstoffatomen, die substituiert sein kann durch 1, 2 oder 3 Hydroxy-, Carboxy-, Carbalkoxy- mit 1 bis 4 Kohlenstoffatomen, Amino- oder Mono- oder Dialkylaminoreste mit 1 bis 4 Kohlenstoffatomen oder durch Chlor- oder Fluoratome; den Rest einer Cycloalkan-Alkansäure, deren Alkylkette 2 bis 4 Kohlenstoffatome, und deren Cycloalkanteil 5 bis 6 Ringkohlenstoffatome enthält; einen benzoylischen Rest, der mono-, di- oder trisubstituiert sein kann durch Hydroxy-, Alkyloxy- mit 1 bis 4 Kohlenstoffatomen, Carboxy-, Carbalkoxy- mit 1 bis 4 Kohlenstoffatomen, Alkanoyl- mit 1 bis 4 Kohlenstoffatomen, Alkanoyloxy- mit 1 bis 4 Kohlenstoffatomen, Nitro-, Amino- oder Mono- oder Dialkylaminoreste mit 1 bis 4 Kohlenstoffatomen oder durch Chlor- oder Fluoratome; den Rest einer Phenylalkansäure, deren aliphatische Kette 1 bis 4 Kohlenstoffatome aufweist, und deren Phenylrest substituiert sein kann durch einen oder zwei Hydroxy-, Alkyloxy- mit 1 bis 4 Kohlenstoffatomen, Amino-, Carboxy-, Carbalkoxy- mit 1 bis 4 Kohlenstoffatomen, Alkanoyl- mit 1 bis 4 Kohlenstoffatomen oder Alkanoyloxyreste mit 1 bis 4 Kohlenstoffatomen oder Chlor- oder Fluoratome; einen Nikotinsäure- oder Isonikotinsäurerest; einen Alkyloxycarbonylrest mit 1 bis 4 Kohlenstoffatomen; einen Carbamoylrest, der monosubstituiert sein kann durch einen Alkylrest mit 1 bis 4 Kohlenstoffatomen oder einen Phenylrest; oder einen Rest einer nichtsubstituierten Phosphorsäure oder einer Dimethyl-, Diäthyl- oder Monohydroxyphenylphosphorsäure, bedeutet, zur Herstellung von pharmazeutischen Zusammensetzungen für die Behandlung von Säugetiererkrankungen, bei denen eine Änderung der Immunantwort des Organismus auftritt, oder zur Behandlung von Säugetiererkrankungen aus der Gruppe bakterielle Viruserkrankungen, parasitäre Viruserkrankungen, Krebserkrankungen und Autoimmunerkrankungen.

2. Verwendung nach Anspruch 1, dadurch gekennzeichnet, dass R in den Derivaten der allgemeinen Formel I bedeutet: einen Alkylrest mit 1 bis 4 Kohlenstoffatomen der substituiert sein kann durch einen Hydroxy-, Alkyloxy- mit 1 bis 4 Kohlenstoffatomen, Amino- oder Nitrilrest; einen Cycloalkanalkylrest, dessen Alkylteil 1 bis 4 Kohlenstoffatome, und dessen Ring 5 bis 6 Kohlenstoffatome aufweist; einen Benzyl-, Phenyläthyl- oder Phenylpropylrest, nichtsubstituiert; einen Tetrahydropyranylrest; einen Rest einer Alkancarbonsäure mit 3 bis 18 Kohlenstoffatomen, die substituiert sein kann durch einen Hydroxy- oder Carboxyrest; einen Rest einer Cycloalkancarbonsäure mit 5 bis 6 Ringkohlenstoffatomen, die substituiert sein kann durch einen benzoylischen Rest, der seinerseits mono-, di- oder trisubstituiert sein kann durch Hydroxy-, Carboxy-, Carbalkoxy- mit 1 bis 4 Kohlenstoffatomen oder Alkanoyloxyreste mit 1 bis 4 Kohlenstoffatomen oder durch Chlor- oder Fluoratome; den Rest einer Phenylalkansäure, deren aliphatische Kette 1 bis 4 Kohlenstoffatome enthält und deren Phenylteil nichtsubstituiert ist; den Rest einer Nikotin- oder Isonikotinsäure; einen Alkyloxycarbonylrest mit 1 bis 4 Kohlenstoffatomen; einen Carbamoylrest, der monosubstituiert ist durch einen Alkylrest mit 1 bis 4 Kohlenstoffatomen oder den Phenylrest; oder einen Rest einer nichtsubstituierten Phosphorsäure oder einer Dimethyl-, Diäthyl- oder Monohydroxyphenylphosphorsäure.

3. Verwendung nach Anspruch 1, dadurch gekennzeichnet, dass R in den Derivaten der allgemeinen Formel I

bedeutet: einen Rest einer Alkancarbonsäure mit 3 bis 18 Kohlenstoffatomen, die substituiert sein kann durch einen Hydroxy- oder Carboxyrest; einen Rest einer Cycloalkancarbonsäure mit 5 bis 6 Ringkohlenstoffatomen, die substituiert sein kann durch einen Carboxyrest; einen benzoylischen Rest, der mono-, di- oder trisubstituiert sein kann durch Hydroxy-, Alkyloxy- mit 1 bis 4 Kohlenstoffatomen, Carboxy-, Carbalkoxy- mit 1 bis 4 Kohlenstoffatomen oder Alkanoyloxyreste mit 1 bis 4 Kohlenstoffatomen oder durch Chlor- oder Fluoratome; einen Rest einer Phenylalkansäure, deren aliphatische Kette 1 bis 4 Kohlenstoffatome aufweist, und deren Phenylteil nichtsubstituiert ist; einen Rest einer Nikotin- oder Isonikotinsäure; einen Alkyloxycarbonylrest mit 1 bis 4 Kohlenstoffatomen; einen Carbamoylrest, der monosubstituiert ist durch eine Alkylgruppe mit 1 bis 4 Kohlenstoffatomen oder durch den Phenylrest; oder einen Rest einer nichtsubstituierten Phosphorsäure oder einer Dimethyl-, Diäthyl- oder Monohydroxyphenylphosphorsäure.

4. Verwendung nach Anspruch 1, dadurch gekennzeichnet, dass R in den Derivaten der allgemeinen Formel I einen Palmitoylrest bedeutet.

5. Verwendung nach Anspruch 1, dadurch gekennzeichnet, dass R in den Derivaten der allgemeinen Formel I einen Heptylrest bedeutet.

## Claims

1. Use of a 3-O-substituted derivative of (+)-cyanidan-3-ol of the general formula

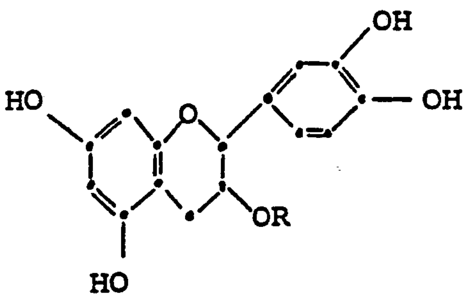

I

wherein R is a $C_1$-$C_4$ alkyl radical which may be substituted by one or two of the groups selected from hydroxy, $C_1$-$C_4$-alkoxy, carboxy, $C_1$-$C_4$ alkanoyloxy, amino mono- or di($C_1$-$C_4$) alkylamino or cyano; $C_5$-$C_{14}$ alkyl, a cycloalkyl radical containing 5 or 6 ring carbon atoms which may be substituted by one or two of the groups selected from hydroxy, $C_1$-$C_4$ alkoxy, carboxy, $C_1$-$C_4$ alkanoyloxy, amino or di($C_1$-$C_4$)alkylamino; a cycloalkylalkyl radical the alkyl moiety of which contains 1 to 4 carbon atoms and which contains 5 to 6 carbon atoms in the ring; a phenyl radical which may carry one, two or three substituents selected from hydroxy, methoxy, ethoxy, propoxy, amino and nitro; a benzyl, phenylethyl or phenylpropyl radical which may be mono- or disubstituted at the benzene ring by one or two of the groups selected from hydroxy, methoxy, ethoxy, propoxy, carboxy, $C_1$-$C_4$ carbalkoxy, nitro, amino or mono- or di($C_1$-$C_4$) alkylamino or by chlorine or fluorine atoms; a tetrahydropyranyl radical; a pyridyl radical which may be substituted by a nitro, amino or hydroxy group; the radical of an alkanecarboxylic acid containing from 3 to 18 carbon atoms which may be substituted by one or more of the groups selected from hydroxy, carboxy, $C_1$-$C_6$ carbalkoxy, $C_1$-$C_5$ alkanoyl, amino or mono- or di($C_1$-$C_4$) alkylamino or by chlorine or fluorine atoms; the radical of a cycloalkanecarboxylic acid containing 5 or 6 ring carbon atoms which may be substituted by one, two or three of the groups hydroxy, carboxy, $C_1$-$C_4$ carbalkoxy, amino or mono- or di($C_1$-$C_4$) alkylamino or by chlorine or fluorine atoms; the radical of a cycloalkylalkanoic acid containing 2 to 4 carbon atoms in the alkyl chain and containing 5 or 6 ring carbon atoms in the cycloalkane moiety; a benzoyl radical which may be substituted by one, two or three members of the group consisting of hydroxy, $C_1$-$C_4$ alkoxy, carboxy, $C_1$-$C_4$ carbalkoxy, $C_1$ -$C_4$ alkanoyl, $C_1$-$C_4$ alkanoyloxy, nitro, amino or mono- or di($C_1$-$C_4$) alkylamino or by chlorine or fluorine atoms; the radical of a phenylalkanoic acid containing 1 to 4 carbon atoms in the aliphatic chain and the phenyl group of which may be substituted by one or two of the groups hydroxy, $C_1$-$C_4$ alkoxy, amino, carboxy, $C_1$-$C_4$ carbalkoxy, $C_1$ -$C_4$ alkanoyl, $C_1$-$C_4$ alkanoyloxy or by chlorine or fluorine atoms; the radical of a nicotinic or isonicotinic acid; a $C_1$-$C_4$ alkoxycarbonyl radical; a carbamoyl radical which may be substituted by a $C_1$-$C_4$ alkyl group or phenyl; or a radical of an unsubstituted phosphoric acid or of a dimethyl-, diethyl- or monohydroxyphenylphosphoric acid, for the preparation of pharmaceutical compositions for the treatment of illnesses in mammals in which a change in the immune response of the organism occurs, or for the treatment of illnesses in mammals selected from the group consisting of bacterial viral diseases, parasitic viral diseases, carcinoses and auto-immune diseases.

2. Use according to claim 1, wherein R in the derivatives of the general formula I is $C_1$-$C_4$ alkyl which may be substituted by hydroxy, $C_1$-$C_4$ alkoxy, amino or cyano, a cycloalkanealkyl radical containing 1 to 4 carbon atoms in the alkyl moiety and 5 to 6 carbon atoms in the ring, an unsubstituted benzyl, phenylethyl or phenylpropyl radical; a tetrahydropyranyl radical; a radical of an alkanecarboxylic acid containing from 3 to 18 carbon atoms which may be substituted by hydroxy or carboxy; the radical of a cycloalkanecarboxylic acid containing 5 or 6

ring carbon atoms which may be substituted by a benzyl radical which may in turn be substituted by one, two or three members selected from the group consisting of hydroxy, carboxy, alkoxycarbonyl containing from 1 to 4 carbon atoms or $C_1$-$C_4$ alkanoyloxy or by chlorine or fluorine atoms; the radical of a phenylalkanoic acid containing 1 to 4 carbon atoms in the aliphatic chain and the phenyl group of which is unsubstituted; a radical of a nicotinic or isonicotinic acid; a $C_1$-$C_4$ alkoxycarbonyl radical; a carbamoyl radical which is substituted by a $C_1$-$C_4$ alkyl group or phenyl; or the radical of an unsubstituted phosphoric acid or of a dimethyl-, diethyl- or monohydroxyphenylphosphoric acid.

3. Use according to claim 1, wherein R in the derivatives of the formula I is a radical of an alkanecarboxylic acid containing from 3 to 18 carbon atoms which may be substituted by hydroxy or carboxy; a radical of a cycloalkanecarboxylic acid containing 5 or 6 ring carbon atoms which may be substituted by a carboxy group, a benzoyl group which may be substituted by one, two or three members selected from the group consisting of hydroxy, $C_1$-$C_4$ alkoxy, carboxy, $C_1$-$C_4$ carbalkoxy, or $C_1$-$C_4$ alkanoyloxy or by chlorine or fluorine atoms; the radical of a phenylalkanoic acid containing 1 to 4 carbon atoms in the aliphatic chain and the phenyl group of which is unsubstituted; the radical of a nicotinic or isonicotinic acid; a $C_1$-$C_4$ alkoxycarbonyl radical; a carbamoyl radical which is substituted by $C_1$-$C_4$ alkyl group or phenyl; or a radical of an unsubstituted phosphoric acid or of a dimethyl-, diethyl- or monohydroxyphenylphosphoric acid.

4. Use according to claim 1, wherein R in the derivatives of the general formula I is a palmitoyl radical.

5. Use according to claim 1, wherein R in the derivatives of the general formula I is a heptyl radical.

**Revendications**

1. Application d'un dérivé 3-O-substitué de (+)-cyanidanol-3 de formule générale

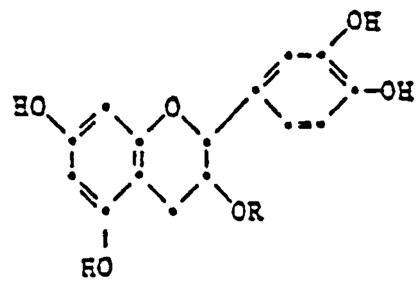

I

et leurs sels pharmaceutiquement acceptables, dans lesquels R représente un radical alcoyle ayant de 1 à 4 atomes de carbone, qui peut être substitué par un ou deux radicaux hydroxy, alcoyloxy en C1 à C4, carboxy, alcanoyloxy en C1 à C4, amino, mono- ou dialcoylamino ayant de 1 à 4 atomes de carbone dans la fraction alcoyle ou nitrile; un radical alcoyle en C5 à C14; un radical cycloalcoyle ayant de 5 à 6 atomes de carbone dans son noyau, qui peut être substitué par un ou deux radicaux hydroxy, alcoyloxy en C1 à C4, carboxy, alcanoyloxy en C1 à C4, amino ou dialcoyle en C1 à C4-amino; un radical cycloalcanalcoyle dont la fraction alcoyle présente de 1 à 4 atomes de carbone et dont le noyau présente 5 à 6 atomes de carbone; un radical phényle, qui peut être mono-, diou trisubstitué par des radicaux hydroxy, méthoxy, éthoxy, propoxy, amino ou nitro; un radical benzyle, phényléthyle ou phénylpropyle, qui peut être mono- ou disubstitué sur le noyau benzène par un ou deux radicaux hydroxy, éthoxy, méthoxy, propoxy, carboxy, carbalcoxy en C1 à C4, nitro, amino ou mono- ou dialcoylamino ayant de 1 à 4 atomes de carbone dans la fraction alcoyle, ou par des atomes de chlore ou de fluor; un radical tétrahydropyranyle; un radical pyridyle, qui peut être substitué par un radical nitro, amino ou hydroxy; le radical d'un acide alcanecarboxylique en C3 à C18, qui peut être substitué par un ou plusieurs radicaux hydroxy, carboxy, carbalcoxy en C1 à C6, alcanoyle en C1 à C5, amino ou mono- ou dialcoylamino ayant de 1 à 4 atomes de carbone dans la fraction alcoyle ou par des atomes de chlore ou de fluor; le radical d'un acide cycloalcanecarboxylique ayant de 5 à 6 atomes de carbone dans son noyau, qui peut être substitué par 1, 2 ou 3 radicaux hydroxy, carboxy, carbalcoxy ayant de 1 à 4 atomes de carbone, amino ou mono- ou dialcoylamino ayant de 1 à 4 atomes de carbone ou par des atomes de chlore ou de fluor; le radical d'un acide cycloalcane-alcanoïque, dont la chaîne alcoyle contient de 2 à 4 atomes de carbone, et dont la fraction cycloalcane contient de 5 à 6 atomes de carbone dans son noyau; un radical benzoylique, qui peut être mono-, di- ou trisubstitué par des radicaux hydroxy, alcoyloxy en C1 à C4, carboxy, carbalcoxy en C1 à C4, alcanoyle en C1 à C4, alcanoyloxy en C1 à C4, nitro, amino ou mono- ou dialcoylamino ayant de 1 à 4 atomes de carbone ou par des atomes de chlore ou de fluor; le radical d'un acide phénylalcanoïque, dont la chaîne aliphatique présente de 1 à 4 atomes de carbone, et dont le radical phényle peut être substitué par un ou deux radicaux hydroxy, alcoyloxy en C1 à C4, amino, carboxy, carbalcoxy en C1 à C4, alcanoyle en C1 à C4 ou des radicaux alcanoyloxy en C1 à C4 ou des atomes de chlore ou de fluor; un radical acide nicotinique ou isonicotinique; un radical alcoyloxycarbonyle en C1 à C4; un radical carbamoyle, qui peut être monosubstitué par un radical alcoyle en C1 à C4 ou un radical phényle; ou un radical d'un acide phosphorique non substitué ou d'un acide diméthyl-, diéthyl- ou monohydroxyphénylphosphorique,

à la préparation de préparations pharmaceutiques pour le traitement de maladies des mammifères dans lesquelles apparaît une modification de la réponse immune de l'organisme, ou au traitement des maladies de mammifères du groupe des maladies virales bactériennes, des maladies virales parasitaires, des maladies cancéreuses et des maladies autoimmunes.

2. Application selon la revendication 1, caractérisée en ce que R dans les dérivés de formule générale I représente: un radical alcoyle en C1 à C4, qui peut être substitué par un radical hydroxy, alcoyloxy en C1 à C4, amino ou nitrile; un radical cycloalcanalcoyle dont la fraction alcoyle présente de 1 à 4 atomes de carbone et dont le noyau présente de 5 à 6 atomes de carbone; un radical benzyle, phényléthyle ou phénylpropyle non substitué; un radical tétrahydropyranyle; un radical d'un acide alcanecarboxylique en C3 à C18, qui peut être substitué par un radical hydroxy ou carboxy; un radical d'un acide cycloalcanecarboxylique ayant de 5 à 6 atomes de carbone dans son noyau, qui peut être substitué par un radical benzoylique, qui de son côté peut être mono-, di- ou trisubstitué par des radicaux hydroxy, carboxy, carbalcoxy en C1 à C4 ou des radicaux alcanoyloxy en C1 à C4 ou par des atomes de chlore ou de fluor; le radical d'un acide phénylalcanoïque dont la chaîne aliphatique contient de 1 à 4 atomes de carbone et dont la fraction phényle est non substituée; le radical d'un acide nicotinique ou isonicotinique; un radical alcoyloxycarbonyle en C1 à C4; un radical carbamoyle, qui est monosubstitué par un radical alcoyle en C1 à C4 ou le radical phényle; ou un radical d'un acide phosphorique non substitué ou d'un acide diméthyl-, diéthyl- ou monohydroxyphénylphosphorique.

3. Application selon la revendication 1, caractérisée en ce que R dans les dérivés de formule générale I représente: un radical d'un acide alcanecarboxylique en C3 à C18, qui peut être substitué par un radical hydroxy ou carboxy: un radical d'un acide cycloalcanecarboxylique ayant de 5 à 6 atomes de carbone dans son noyau, qui peut être substitué par un radical carboxy; un radical benzoylique, qui peut être mono-, di- ou trisubstitué par des radicaux hydroxy, alcoyloxy en C1 à C4, carboxy, carbalcoxy en C1 à C4 ou des radicaux alcanoyloxy en C1 à C4 ou par des atomes de chlore ou de fluor; un radical d'un acide phénylalcanoïque dont la chaîne aliphatique présente de 1 à 4 atomes de carbone et dont la fraction phényle n'est pas substituée; un radical d'un acide nicotinique ou isonicotinique; un radical alcoyloxycarbonyle en C1 à C4; un radical carbamoyle, qui est monosubstitué par un groupe alcoyle en C1 à C4 ou par le radical phényle; ou un radical d'un acide phosphorique non substitué ou d'un acide diméthyl-, diéthyl- ou monohydroxyphénylphosphorique.

4. Application selon la revendication 1, caractérisée en ce que R dans les dérivés de formule générale I représente un radical palmitoyle.

5. Application selon la revendication 1, caractérisée en ce que R dans les dérivés de formule générale I représente un radical heptyle.